# EUROPEAN PATENT SPECIFICATION

(11) **EP 3 254 621 B1**
(45) Date of publication and mention of the grant of the patent: **22.07.2020**
(21) Application number: 16203554.7
(22) Date of filing: 12.12.2016
(51) Int. Cl.: A61B 6/00, A61B 34/30

(54) **3D IMAGE SPECIAL CALIBRATOR, SURGICAL LOCALIZING SYSTEM AND METHOD**
SPEZIALKALIBRATOR FÜR 3D-BILDER, CHIRURGISCHES ORTUNGSSYSTEM UND VERFAHREN
ÉTALONNEUR SPÉCIAL D'IMAGE 3D, PROCÉDÉ ET SYSTÈME DE LOCALISATION CHIRURGICALE

(30) Priority: 08.06.2016 CN 201610403984
(43) Date of publication of application: 13.12.2017
(73) Proprietor: Tinavi Medical Technologies Co., Ltd., Beijing 100192 (CN)
(72) Inventor: ZHANG, Songgen, Beijing, Beijing 100192 (CN); TIAN, Wei, Beijing, Beijing 100192 (CN); LIU, Yajun, Beijing, Beijing 100192 (CN); XU, Jin, Beijing, Beijing 100192 (CN); ZHANG, Weijun, Beijing, Beijing 100192 (CN); WANG, Binbin, Beijing, Beijing 100192 (CN)
(74) Representative: Littolff, Denis

(56) References cited:
- EP-A1- 3 009 096
- EP-A2- 0 919 203
- US-A1- 2005 281 385
- US-A1- 2015 343 239

## Description

### TECHNICAL FIELD

The present invention relates to a calibrator for 3D image, a surgical positioning system, and a method, which belong to the technical field of surgical navigation.

### RELATED ART

With the widespread application of minimally invasive surgery and the continuously increasing requirements for the precision of positioning of instruments or implants in surgery in recent years, medical image guidance-based auxiliary positioning or surgical navigation products have made great progress. The implementation of such products generally includes several steps: first, space calibrating and image registration, that is, a spatial transformation relationship between coordinate systems of a surgical target (patient), images of the target, and an auxiliary positioning device is calculated by using a spatial measurement device calibrating method. The step generally is referred to as multi-coordinate system calibration or image registration. The next step is surgical planning and guidance, that is, a preoperative or intraoperative image having an accurate calibration is displayed, and a doctor plans a surgery path on the image or on a re-constructed 3D model. Subsequently, the next step is surgical implementation, which mainly involves surgery path positioning, that is, guiding a doctor to place a surgical tool guiding device onto the surgery path by hands or to directly control an execution mechanism such as a robotic arm, to accurately place a guiding device onto the surgery path, so as to guarantee the precision of surgery path guidance, and the doctor implements operations, such as surgical instruments implantation, by means of the guiding device.

For example, US 2005/281385 A1 discloses a method and system for improved correction of registration error in a fluoroscopic image. Among the foregoing steps, the step of spatial calibrating and image registration is an extremely significant step. The step generally means unifying a plurality of coordinate systems (generally including an image coordinate system, a tool (auxiliary positioning apparatus) coordinate system, and a patient coordinate system) into one same coordinate system in an image guidance-based surgical positioning system. The process is also referred to as registration or calibration. The precision of the registration determines the precision of the auxiliary positioning or surgical navigation.

According to types of medical images used (fluoroscopy images, or CT images) and sources of the medical images (preoperative images, or intraoperative images obtained on site), the commonly used image registration methods at present are as follows:
Scenario 1: the requirement for image registration is "obtaining 3D images before a surgery and doing images registration during the surgery".
   A method for image registration that meets the requirement for image guided surgery includes: ① During a surgery, positions of some easy identified anatomical feature points of a human body are collected by a spatial measurement device. The positions are paired with corresponding feature points in an image, to implement image registration. ② During a surgery, point-cloud of a feature contour part on the human body is continuously obtained by using a spatial tracking and measuring device, and the point-cloud is paired with corresponding position shapes in preoperative images, to implement image registration. ③ Preoperative 3D images of patient is obtained with several markers attached on patient. During the surgery, positions of those markers are obtained by using the spatial measurement device, meanwhile, corresponding marker in the image are paired and marked to implement image registration.
Scenario 2: the requirement for image registration is "obtaining 3D images before a surgery and spatial calibrating them with fluoroscopy images obtained during the surgery".
   A method to meet the requirement for image registration includes: matching a contour or an edge shape of an anatomical structure identified in the intraoperative fluoroscopy images obtained from different angle with the preoperative 3D image by using a special algorithm, to implement registration.
Scenario 3: the requirement for image registration is "obtaining a 2D fluoroscopy image during a surgery and registering on site".
   A method for image registration that meets the requirement includes: a patient tracker and a robot tracker that are traced by a spatial measurement device, the patient tracker is fixedly mounted on a patient body; a dual-parallel, planar-structured special calibrator is mounted at the terminal end of a robotic arm; the robot tracker is mounted on the robotic arm; and during a surgery, fluoroscopy images are obtained from at least two different angles, and intraoperative fluoroscopy image registration is implemented by identifying calibrator marked points in the image.
Scenario 4: the requirement for image registration is "obtaining a set of 3D images during the surgery and doing image registration on site".

A method that meets the requirement includes: position of an intraoperative 3D imaging device (CT or MRI or C-arm with 3D option) and position of patient are obtained by trackers installed on the imaging device and on the patient body (or place fixed relative to the patient body). A spatial transform relationship (a rotation and translation matrix) between the intraoperative 3D image coordinate system and the patient coordinate system is calculated by calibrating or by means of parameters in an imaging device provided by the imaging device manufacture, to implement intraoperative 3D image registration.

The method in scenario 4 depends on a tracker mounted on an intraoperative imaging device, and meanwhile a series of imaging parameters of the imaging device need to be calibrated in advance; and therefore, the method is not easy to implement.

### SUMMARY

With respect to the above problem, an objective of the present invention is providing a surgical positioning system and a positioning method. The positioning method is capable of implementing automatically intraoperative 3D image registration while not depending on parameters of a 3D imaging device, and is easy to implement.

To achieve the objective, the present invention adopts the following technical solution: a calibrator for 3D image, characterized in that: the calibrator for 3D image includes a calibrator plane and a calibrator handle, wherein the calibrator plane is flat or arc-shaped, and at least four marked points to be identified by a 3D imaging device are arranged on the calibrator plane; and one end of the calibrator handle is fixedly connected to the calibrator plane, and the other end of the calibrator handle is provided with a connector for connecting a surgical robotic arm.

An arrangement shape of all the marked points located on the calibrator plane has an anisotropy.

The calibrator plane is made of an X-ray transparent material; and the marked points are made of an X-ray opaque material.

A surgical positioning system, characterized in that: comprising a surgical robot, a host computer, a spatial measurement device, a robot tracker, a patient tracker, a 3D imaging device, and any of the above calibrators for 3D image ; the surgical robot is a robotic arm having at least three translational degrees of freedom and three rotational degrees of freedom; the host computer is electrically connected to the surgical robot so as to control a motion of the surgical robot; the calibrator for 3D image and the robot tracker are detachably connected to the terminal end of the surgical robot; the patient tracker is fixed on the body of a patient; the spatial measurement device is used to measure position and orientation of the robot tracker and the patient tracker and transmit position data to the host computer; the 3D imaging device is configured to scan the calibrator for 3D image and the patient at the same time and transmit images to the host computer; and the host computer performs corresponding identification of the marked points in the image and marked points on the calibrator for 3D image.

The surgical positioning system further comprises a guiding device, wherein the guiding device is detachably connected to the terminal end of the surgical robot.

A positioning method includes the following steps: 1) placing a calibrator for 3D image, installed on the surgical robot, closing to surface of the patient body; scanning both the calibrator and the patient by using a 3D imaging device; transmitting 3D images with marked points on the calibrator and the patient to the host computer; meanwhile, the spatial measurement device tracking coordinates of the robot tracker and the patient tracker, and transmitting the coordinates to the host computer; 2) cyclically comparing, by the host computer, geometric features between the marked points in the image and preset pattern of these marked points, to implement corresponding identification of the marked points on the calibrator for 3D image and those in the image; 3) calculating, by the host computer, a coordinate transformation relationship between the patient image and the robot tracker by means of a given coordinate relationship between the marked points on the calibrator for 3D image and the robot tracker, and further calculating a coordinate transformation relationship between the patient image and the surgical robot; and 4) calculating, according to the coordinate transformation relationship between the patient image and the surgical robot, coordinates of a spatial point that corresponds to any point in the patient image, in a robot coordinate system, and further calculating coordinates of a surgery path that is determined in the patient image, in the robot coordinate system.

In step 2), the specific process of identification between marked points on the calibrator for 3D image and the marked points in the image is as follows: ① grouping the marked points on the calibrator into a group A and a group B, wherein each group includes three or more marked points; ② reading information about the marked points included in the group A and the group B in step ① and information about the calibrator for 3D image 1, and reading the images obtained by scanning in step 1); ③ performing threshold segmentation on the images obtained in step ② and extracting and generating valid polygon; ④ fitting and determining, according to the information about the calibrator for 3D image obtained in step ②, the polygon obtained in step ③, so as to screen to obtain marked points in the image; ⑤ calculating a distance between each two marked points among the marked points in the image obtained in step ④; ⑥ selecting three marked points from calibrator marked points in the group A to construct a triangle as a triangular template, and searching for a triangle that is approximately, identically equal to the triangular template in the image; if no such triangle is found, selecting three marked points from calibrator marked points in the group B to construct a triangle as a triangular template, and searching for a triangle that is approximately, identically equal to the triangular template in the image; if still no such triangle is found, selecting calibrator marked points from the group A and the group B to construct a triangle as a triangular template, and searching for a triangle that is approximately, identically equal to the triangular template in the image; and ⑦ retaining vertex numbers of the pair of congruent triangles according to a one-to-one correspondence, to form a match point pair, and searching for a corresponding image marked point of a calibrator marked point apart from the triangular template, in the image by using the congruent triangular template as a reference, until all image marked points match the calibrator marked points.

The present invention adopts the foregoing technical solutions, and therefore has the following advantages: the present invention implements high-precision fusion or registration of a patient coordinate system, an image coordinate system, and a robot coordinate system, by using a calibrator for 3D image and by means of a spatial measurement device, a patient tracker, and a robot tracker, and performs point pair identification and marking without manual participating, thereby having a high automation degree, not depending on a special support of a 3D imaging device, and having a wide applicability.

### BRIEF DESCRIPTION OF THE DRAWINGS

Fig. 1 is a schematic structural diagram of a calibrator for 3D image;
Fig. 2 is a schematic structural diagram of a surgical positioning system of the present invention; and

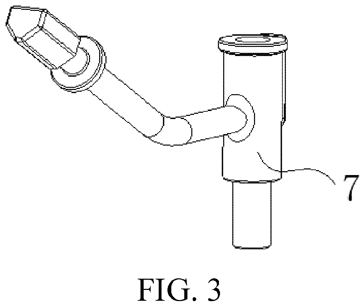

Fig. 3 is a schematic structural diagram of a guiding device of the present invention.

### DETAILED DESCRIPTION

The present invention is described in detail below in the embodiments in combination with the accompanying drawings.

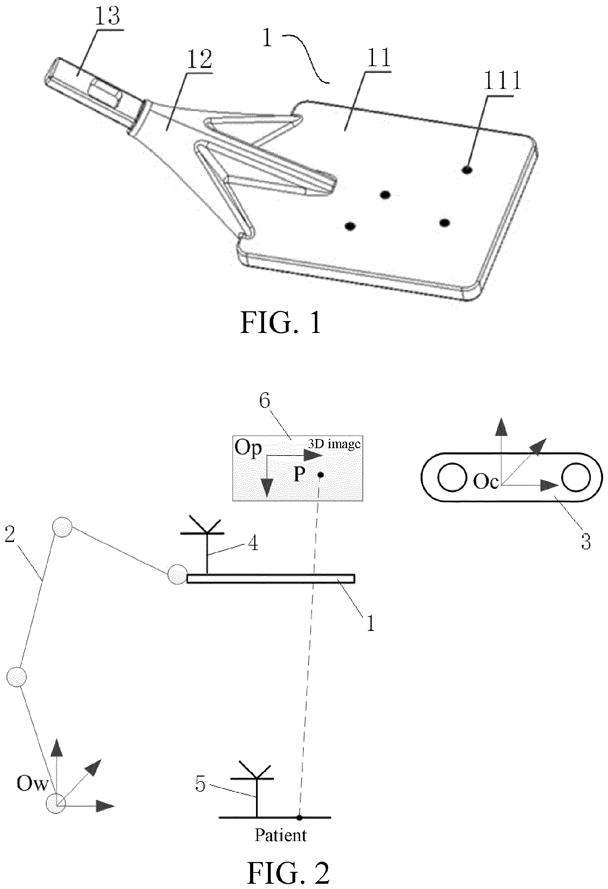

As shown in Fig. 1, a calibrator for 3D image 1 is provided. The calibrator for 3D image 1 includes a calibrator plane 11 and a calibrator handle 12. The calibrator plane 11 is flat or arc-shaped. At least four marked points 111 are arranged on the calibrator plane 1. The marked points 111 are configured to be identified and scanned by a 3D imaging device to form an image. One end of the calibrator handle 12 is fixedly connected to the calibrator plane 11, and the other end of the calibrator handle 12 is provided with a connector 13 for connecting to the surgical robotic arm.

Further, an arrangement shape of all the marked points 111 located on the calibrator plane 1 has an anisotropy (for example, distances between any two marked points 111 are not equal).

Further, the calibrator plane 1 is made of an X-ray transparent material; and the marked points 111 are made of an X-ray opaque material.

As shown in Fig. 2, on the basis of the calibrator for 3D image 1, the present invention further provides a surgical positioning system. The surgical positioning system includes a calibrator for 3D image 1, a surgical robot 2, a host computer (not shown), a spatial measurement devices spotter 3, a robot tracker 4, a patient tracker 5, a 3D imaging device 6, and a guiding device 7. The surgical robot 2 is a robotic arm having at least three translational degrees of freedom and three rotational degrees of freedom. The host computer is electrically connected to the surgical robot 2 so as to control a motion of the surgical robot 2. The calibrator for 3D image 1 and the robot tracker 4 are connected to a terminal end of the surgical robot by means of a quick-mount and quick-release mechanism. The patient tracker 5 is fixed on the body of a patient. The spatial measurement devices spotter 3 can measure spatial measurement devices of the robot tracker 4 and the patient tracker 5, and refreshes the coordinates at a certain frequency, to implement real-time tracing. The spatial measurement devices spotter 3 can adopt a stereo vision principle-based high-precision optic tracing camera or may be based on other principles, and transmit position data to the host computer. The 3D imaging device 6 is configured to scan the calibrator for 3D image 1 so as to image the marked points 111, and the host computer performs corresponding identification on the marked points in the image and the marked points 111 on the calibrator for 3D image 1. The guiding device 7 is an apparatus for fixing a needle insertion path. The guiding device 7 is connected to the surgical robot 2 by means of a quick-mount and quick-release structure same as the calibrator 1, and is mounted for use alternately with the calibrator for 3D image 1 according to needs in a surgery.

The present invention preferably adopts a cone-beam CT machine (CBCT machine) as a 3D imaging device.

On the basis of the above positioning system, the present invention provides a positioning method, which is applicable to spatial positioning of a surgery path. The method includes the following steps:
1) placing a calibrator for 3D image 1, installed on the surgical robot 2, closing to surface of the patient body (close to but not in contact); scanning both the calibrator for 3D image 1 and the patient by using a 3D imaging device 6 (only one 3D image scanning is needed without fluoroscopy from a plurality of different angles for several times); transmitting 3D images with marked points 111 on the calibrator 1 and the patient to the host computer; meanwhile, the spatial measurement devices spotter 3 tracking coordinates of the robot tracker 4 and the patient tracker 5, and transmitting the coordinates to the host computer.
2) cyclically comparing, by the host computer, geometric features between the marked points in the image and preset pattern of these marked points, to implement corresponding identification of the marked points 111 on the calibrator for 3D image 1 and those in the image.
3) calculating, by the host computer, a coordinate transformation relationship between the patient image and the robot tracker 4 by means of a given coordinate relationship between the marked points 111 on the calibrator for 3D image 1 and the robot tracker 4 (it should be noted that the host computer may further calculate the coordinate transformation relationship between the patient image and the patient tracker 5 according to coordinates of the robot tracker 4 and the patient tracker 5 obtained by the spatial measurement devices spotter 3), and further calculating a coordinate transformation relationship between the patient image and the surgical robot 2. The step may also be directly calculating, by the host computer, a coordinate transformation relationship between the patient image and the surgical robot 2 according to a given coordinate relationship between the marked points 111 on the calibrator for 3D image 1 and the surgical robot 2.
4) Calculating, according to the coordinate transformation relationship between the patient image and the surgical robot 2 obtained in step 3), coordinates of a spatial point that corresponds to any point in the patient image, in a robot coordinate system. If the surgery path is represented by a straight line in the patient image, coordinates of the surgery path in the robot coordinate system can be calculated.

By means of special software, a doctor may draw, according to treatment needs, a surgery path on an image after registration, and may control the surgical robot 2 to move accurately after calculating spatial measurement devices of the surgery path by means of the spatial positioning method for the surgery path, so as to enable a guiding structure of the guiding device 7 that is connected to the terminal end of the surgery robot 2 to orient at the surgery path. In the foregoing process, the spatial measurement devices spotter 3 having a real-time tracing function monitors the patient tracker 5 (that is, a motion of the patient) in real time, and calculates an orientation and magnitude of the motion. The surgical robot 2 may modify its own motion according to data such as the orientation and magnitude of the motion, so as to guarantee that the guiding device precisely conforms to the planned surgery path.

In step 2), the specific process of identification between marked points 111 on the calibrator for 3D image 1 and the marked points in the image is as follows:
① grouping the marked points 111 on the calibrator for 3D image 1 into a group A and a group B, wherein each group includes three or more marked points 111;
② reading information about the marked points included in the group A and the group B in step ① and information about the calibrator for 3D image 1, and reading the images obtained by scanning in step 1);
③ performing threshold segmentation on the images obtained in step ② and extracting and generating valid polygon;
④ fitting and determining, according to the information about the calibrator for 3D image 1 obtained in step ②, the polygon obtained in step ③, so as to screen to obtain marked points in the image;
⑤ calculating a distance between each two marked points among the marked points in the image obtained in step ④;
⑥ selecting three marked points from calibrator marked points in the group A to construct a triangle as a triangular template, and searching for a triangle that is approximately, identically equal to the triangular template in the image; if no such triangle is found, selecting three marked points from calibrator marked points in the group B to construct a triangle as a triangular template, and searching for a triangle that is approximately, identically equal to the triangular template in the image; and if still no such triangle is found, selecting calibrator marked points from the group A and the group B to construct a triangle as a triangular template, and searching for a triangle that is approximately, identically equal to the triangular template in the image; and
⑦ retaining vertex numbers of the pair of congruent triangles according to a one-to-one correspondence, to form a match point pair, and searching for a corresponding image marked point of a calibrator marked point apart from the triangular template, in the image by using the congruent triangular template as a reference, until all image marked points match the calibrator marked points.

## Claims

1. A surgical positioning system, wherein the surgical positioning system comprises a surgical robot (2), a host computer, a spatial measurement devices spotter (3), a robot tracker (4), a patient tracker (5), a 3D imaging device (6), and a calibrator for 3D image (1) comprising a calibrator plane (11) and a calibrator handle (12), wherein the calibrator plane (11) is flat or arc-shaped, and at least four marked points (111) to be identified by the 3D imaging device (6) are arranged on the calibrator plane (11); and one end of the calibrator handle (12) is fixedly connected to the calibrator plane (11), and the other end of the calibrator handle (12) is provided with a connector (13) for connecting to a surgical robotic arm; the surgical robot (2) is a robotic arm having at least three translational degrees of freedom and three rotational degrees of freedom; the host computer is electrically connected to the surgical robot (2) so as to control a motion of the surgical robot (2); the calibrator for 3D image (1) and the robot tracker (4) are detachably connectable to the terminal end of the surgical robot (2); the patient tracker (5) is adapted to be fixable on the body of a patient; the spatial measurement device (3) is adapted to measure position and orientation of the robot tracker (4) and the patient tracker (5) and transmit position data to the host computer; the 3D imaging device (6) is configured to scan the calibrator for 3D image and the patient at the same time and transmit images to the host computer; and the host computer is adapted to perform corresponding identification of the marked points in the image and marked points on the calibrator for 3D image (1).

2. The surgical positioning system according to claim 1, wherein the calibrator for 3D image (1) is characterized that an arrangement shape of all the marked points located on the calibrator plane has an anisotropy.

3. The surgical positioning system according to claim 1 or 2, wherein the calibrator for 3D image (1) is characterized that the calibrator plane is made of an X-ray transparent material; and the marked points are made of an X-ray opaque material.

4. The surgical positioning system according to any of the previous claims, **characterized in**: further comprising a guiding device (7), wherein the guiding device (7) is detachably connected to the terminal end of the surgical robot (2).

5. A positioning method implemented using the surgical positioning system according to any of the previous claims, comprising the following steps:
1) placing a calibrator for 3D image (1), installed on the surgical robot (2), close to surface of the patient body; scanning both the calibrator (1) and the patient by using a 3D imaging device (6); transmitting 3D images with marked points on the calibrator (1) and the patient to the host computer; meanwhile, the spatial measurement device (3) tracking coordinates of the robot tracker (4) and the patient tracker (5), and transmitting the coordinates to the host computer;
2) cyclically comparing, by the host computer, geometric features between the marked points in the image and preset pattern of these marked points, to implement corresponding identification of the marked points on the calibrator for 3D image (1) and those in the image;
3) calculating, by the host computer, a coordinate transformation relationship between the patient image and the robot tracker (4) by means of a given coordinate relationship between the marked points on the calibrator for 3D image (1) and the robot tracker (4), and further calculating a coordinate transformation relationship between the patient image and the surgical robot (2); and
4) calculating, according to the coordinate transformation relationship between the patient image and the surgical robot (2), coordinates of a spatial point that corresponds to any point in the patient image, in a robot coordinate system, and further calculating coordinates of a surgery path that is determined in the patient image, in the robot coordinate system.

6. The positioning method according to claim 5, **characterized in that**: in step 2), the specific process of identification between marked points on the calibrator for 3D image (1) and the marked points in the image is as follows:
① grouping the marked points on the calibrator for 3D image (1) into a group A and a group B, wherein each group comprises three or more marked points;
② reading information about the marked points comprised in the group A and the group B in step ① and information about the calibrator for 3D image (1), and reading the images obtained by scanning in step 1);
③ performing threshold segmentation on the images obtained in step ② and extracting and generating valid polygon;
④ fitting and determining, according to the information about the calibrator for 3D image (1) obtained in step ②, the polygon obtained in step ③, so as to screen to obtain marked points in the image;
⑤ calculating a distance between each two marked points among the marked points in the image obtained in step ④;
⑥ selecting three marked points from calibrator marked points in the group A to construct a triangle as a triangular template, and searching for a triangle that is approximately, identically equal to the triangular template in the image; if no such triangle is found, selecting three marked points from calibrator marked points in the group B to construct a triangle as a triangular template, and searching for a triangle that is approximately, identically equal to the triangular template in the image; and if still no such triangle is found, selecting calibrator marked points from the group A and the group B to construct a triangle as a triangular template, and searching for a triangle that is approximately, identically equal to the triangular template in the image; and
⑦ retaining vertex numbers of the pair of congruent triangles according to a one-to-one correspondence, to form a match point pair, and searching for a corresponding image marked point of a calibrator marked point apart from the triangular template, in the image by using the congruent triangular template as a reference, until all image marked points match the calibrator marked points.

## Patentansprüche

1. Chirurgisches Positionierungssystem, wobei das chirurgische Positionierungssystem einen chirurgischen Roboter (2), einen Hostcomputer, eine Erkennungseinrichtung (3) für Vorrichtungen zur räumlichen Messung, eine Roboterverfolgungseinrichtung (4), eine Patientenverfolgungseinrichtung (5), eine 3D-Bildgebungsvorrichtung (6) und eine 3D-Bildkalibrationseinrichtung (1) umfasst, die Kalibrationsfläche (11) und einen Kalibrationseinrichtungsgriff (12) umfasst, wobei die Kalibrationsfläche (11) flach oder bogenförmig ist und wenigstens vier markierte Punkte (111) zum Identifizieren durch die 3D-Bildgebungsvorrichtung (6) an der Kalibrationsfläche (11) angeordnet sind; und wobei ein Ende des Kalibrationseinrichtungsgriffs (12) fest mit der Kalibrationsfläche (11) verbunden ist und das andere Ende des Kalibrationseinrichtungsgriffs (12) mit einem Verbinder (13) zum Verbinden mit einem chirurgischen Roboterarm versehen ist; wobei der chirurgischen Roboter (2) ein Roboterarm mit wenigstens drei translatorischen Freiheitsgraden und Rotationsfreiheitsgraden ist; wobei der Hostcomputer mit dem chirurgischen Roboter (2) elektrisch verbunden ist, um eine Bewegung des chirurgischen Roboters (2) zu steuern; wobei die 3D-Bildkalibrationseinrichtung (1) und die Roboterverfolgungseinrichtung (4) lösbar mit dem Anschlussende des chirurgischen Roboter (2) verbindbar sind; wobei die Patientenverfolgungseinrichtung (5) dazu ausgebildet ist, am Körper eines Patienten fixierbar zu sein; wobei die Vorrichtung (3) zur räumlichen Messung dazu ausgebildet ist, eine Position und Ausrichtung der Roboterverfolgungseinrichtung (4) und der Patientenverfolgungseinrichtung (5) zu messen und Positionsdaten an den Hostcomputer zu übertragen; wobei die 3D-Bildgebungsvorrichtung (6) dazu konfiguriert ist, die 3D-Bildkalibrationseinrichtung und den Patienten zeitgleich abzutasten und Bilder an den Hostcomputer zu übertragen; und der Hostcomputer dazu ausgebildet ist, eine entsprechende Identifizierung der markierten Punkte im Bild und der markierten Punkte an der 3D-Bildkalibrationseinrichtung (1) durchzuführen.

2. Chirurgisches Positionierungssystem nach Anspruch 1, wobei die 3D-Bildkalibrationseinrichtung (1) **dadurch gekennzeichnet ist, dass** eine Anordnungsform aller markierten Punkte an der Kalibrationsfläche eine Anisotropie aufweist.

3. Chirurgisches Positionierungssystem nach Anspruch 1 oder 2, wobei die 3D-Bildkalibrationseinrichtung (1) **dadurch gekennzeichnet ist, dass** die Kalibrationsfläche aus einem röntgendurchlässigen Material hergestellt ist; und die markierten Punkte aus einem röntgenundurchlässigen Material hergestellt sind.

4. Chirurgisches Positionierungssystem nach einem der vorangehenden Ansprüche, ferner **gekennzeichnet durch** eine Führungsvorrichtung (7), wobei die Führungsvorrichtung (7) lösbar mit dem Anschlussende des chirurgischen Roboters (2) verbunden ist.

5. Positionierungsverfahren, das unter Verwendung des chirurgischen Positionierungssystem nach einem der vorangehenden Ansprüche umgesetzt wird, folgende Schritte umfassend:
1) Anordnen einer 3D-Bildkalibrationseinrichtung (1), die am chirurgischen Roboter (2) installiert ist, nahe der Körperoberfläche eines Patienten; Abtasten der Kalibrationseinrichtung (1) und des Patienten unter Verwendung einer 3D-Bildgebungsvorrichtung (6); Übertragen von 3D-Bildern mit markierten Punkten an der Kalibrationseinrichtung (1) und am Patienten an den Hostcomputer; wobei die Vorrichtung (3) zur räumlichen Messung Koordinaten der Roboterverfolgungseinrichtung (4) und der Patientenverfolgungseinrichtung (5) verfolgt und die Koordinaten an den Hostcomputer überträgt;
2) zyklisches Vergleichen geometrischer Merkmale zwischen den markierten Punkten im Bild und einem im Voraus festgelegten Muster dieser markierten Punkte durch den Hostcomputer, um eine entsprechende Identifizierung der markierten Punkte an der 3D-Bildkalibrationseinrichtung (1) und derjenigen im Bild umzusetzen;
3) Berechnen eines Koordinatentransformationsverhältnisses zwischen dem Patientenbild und der Roboterverfolgungseinrichtung (4) durch den Hostcomputer mittels eines gegebenen Koordinatenverhältnisses zwischen den markierten Punkte an der 3D-Bildkalibrationseinrichtung (1) und der Roboterverfolgungseinrichtung (4), und ferner Berechnen eines Koordinatentransformationsverhältnisses zwischen dem Patientenbild und dem chirurgischen Roboter (2); und
4) Berechnen, gemäß dem Koordinatentransformationsverhältnis zwischen dem Patientenbild und dem chirurgischen Roboter (2), von Koordinaten eines räumlichen Punkts in einem Roboterkoordinatensystem, der mit einem beliebigen Punkt im Patientenbild übereinstimmt, und ferner Berechnen von Koordinaten eines Operationswegs im Roboterkoordinatensystem, der in dem Patientenbild bestimmt wird.

6. Positionierungsverfahren nach Anspruch 5, **dadurch gekennzeichnet, dass** in Schritt 2) der spezifische Prozess der Identifizierung zwischen markierten Punkte an der 3D-Bildkalibrationseinrichtung (1) und den markierten Punkten im Bild wie folgt ist:
①. Gruppieren der markierten Punkte an der 3D-Bildkalibrationseinrichtung (1) in eine Gruppe A und eine Gruppe B, wobei jede Gruppe drei oder mehr markierte Punkte umfasst;
②. Auslesen von Informationen zu den markierten Punkten von Gruppe A und Gruppe B in Schritt ① und Informationen zur 3D-Bildkalibrationseinrichtung (1), und Auslesen der in Schritt 1) durch Abtastung erlangten Bilder;
③. Durchführen einer Schwellenwertsegmentierung an den in Schritt ② erlangten Bildern und Extrahieren und Generieren eines gültigen Polygons;
④. Anpassen und Bestimmen des in Schritt ③ erlangten Polygons gemäß den in Schritt ② erlangten Informationen zur 3D-Bildkalibrationseinrichtung (1), um ein Screening zum Erlangen von markierten Punkten im Bild durchzuführen;
⑤. Berechnen eines Abstands zwischen je zwei markierten Punkten unter den markierten Punkten in dem in Schritt ④ erlangten Bild;
⑥. Auswählen von drei markierten Punkten aus den markierten Punkten der Kalibrationseinrichtung in Gruppe A, um ein Dreieck als Dreieckschablone zu konstruieren, und Suchen nach einem Dreieck, das annähernd gleich der Dreieckschablone ist, im Bild; wenn kein solches Dreieck gefunden wird, Auswählen von drei markierten Punkten aus den markierten Punkten der Kalibrationseinrichtung in Gruppe B, um ein Dreieck als Dreieckschablone zu konstruieren, und Suchen nach einem Dreieck, das annähernd gleich der Dreieckschablone ist, im Bild; und wenn weiterhin kein solches Dreieck gefunden wird, Auswählen von markierten Punkten der Kalibrationseinrichtung aus Gruppe A und Gruppe B, um ein Dreieck als Dreieckschablone zu konstruieren, und Suchen nach einem Dreieck, das annähernd gleich der Dreieckschablone ist, im Bild; und
⑦. Beibehalten von Scheitelpunktzahlen des Paars kongruente Dreiecke gemäß einer Eins-zu-eins-Entsprechung, um ein Übereinstimmungspunktepaar zu bilden, und Suchen nach einem entsprechenden markierten Bildpunkt eines markierten Punkts der Kalibrationseinrichtung getrennt von der Dreieckschablone in dem Bild unter Verwendung der kongruenten Dreieckschablone als Referenz, bis alle markierten Bildpunkte mit den markierten Punkten der Kalibrationseinrichtung übereinstimmen.

## Revendications

1. Un système de positionnement chirurgical, dans lequel le système de positionnement chirurgical comprend un robot chirurgical (2), un ordinateur hôte, un dispositif de mesure spatiale (3), un tracker robot (4), un tracker patient (5), un dispositif d'imagerie 3D (6), et un calibreur pour image 3D (1) comprenant un plan de calibreur (11) et une poignée de calibreur (12), dans lequel le plan de calibreur (11) est plat ou arciforme, et au moins quatre points marqués (111) à identifier par le dispositif d'imagerie 3D (6) sont agencés sur le plan de calibreur (11) ; une extrémité de la poignée du calibreur (12) est connectée de façon fixe au plan de calibreur (11), l'autre extrémité de la poignée de calibreur (12) est dotée d'un connecteur (13) pour connecter le bras robotique chirurgical ; le robot chirurgical (2) est un bras robotique doté d'au moins trois degrés translationnels de liberté et trois degrés rotationnels de liberté ; l'ordinateur hôte est connecté électriquement au robot chirurgical (2) afin de contrôler un mouvement du robot chirurgical (2) ; le calibreur pour image 3D (1) et le tracker robot (4) peuvent être connectés de façon détachable à l'extrémité du terminal du robot chirurgical (2) ; le tracker patient (5) a la capacité d'être fixé sur le corps d'un patient ; le dispositif de mesure spatiale (3) a la capacité de mesurer la position et l'orientation du tracker robot (4) et du tracker du patient (5) et transmet les données de position à l'ordinateur hôte ; le dispositif d'imagerie 3D (6) est configuré pour scanner le calibreur pour l'image 3D et le patient en même temps et transmettre les images à l'ordinateur hôte ; l'ordinateur hôte a la capacité d'effectuer l'identification correspondante des points marqués sur l'image et des points marqués sur le calibreur pour image 3D (1).

2. Le système de positionnement chirurgical selon la revendication 1, dans lequel le calibreur pour image 3D (1) est **caractérisé en ce que** un agencement de forme de tous les points marqués situés sur le plan du calibreur dispose d'une anisotropie.

3. Le système de positionnement chirurgical selon la revendication 1 ou 2, dans lequel le calibreur pour image 3D (1) est **caractérisé en ce que** le plan du calibreur est en matériau transparent aux rayons X ; et les points marqués sont en matériau opaque aux rayons X.

4. Le système de positionnement chirurgical selon l'une quelconque des revendications précédentes, **caractérisé en ce que** il comprend en outre un dispositif de guidage (7), dans lequel le dispositif de guidage (7) est connecté de façon détachable à l'extrémité du terminal du robot chirurgical (2).

5. Un procédé de positionnement mettant en œuvre le système de positionnement chirurgical selon l'une quelconque des revendications précédentes, comprenant les étapes suivantes :
1) Le placement d'un calibreur pour image 3D (1), installé sur le robot chirurgical (2), à proximité de la surface du corps du patient ; le scannage du calibreur (1) et du patient à l'aide du dispositif d'imagerie 3D (6) ; la transmission des images 3D avec les points marqués sur le calibreur (1) et le patient à l'ordinateur hôte ; simultanément, le dispositif de mesure spatiale (3) effectue le suivi du tracker robot (4) et du tracker patient (5), et transmet les coordonnées à l'ordinateur hôte ;
2) La comparaison cyclique, par l'ordinateur hôte, des caractéristiques géométriques entre les points marqués et l'image et du modèle prédéfini de ces points marqués, pour effectuer l'identification correspondante des points marqués du calibreur pour image 3D (1) et de ceux de l'image ;
3) La calcul, par l'ordinateur hôte, d'un lien de transformation des coordonnées entre l'image du patient et le tracker robot (4) par moyen d'un lien de coordonnées donné entre les points marqués sur le calibreur pour image 3D (1) et le tracker robot (4), et le calcul supplémentaire d'un lien de transformation des coordonnées entre l'image du patient et le robot chirurgical (2) ; et
4) Le calcul, selon le lien de transformation des coordonnées entre l'image du patient image et le robot chirurgical (2), des coordonnées d'un point spatial qui correspond à un point sur l'image du patient, dans un système de coordonnées du robot, et le calcul supplémentaire des coordonnées d'une procédure chirurgicale qui est déterminée sur l'image du patient, dans le système de coordonnées du robot.

6. Le procédé de positionnement selon la revendication 5, **caractérisé en ce que** à l'étape 2), le processus spécifique d'identification entre les points marqués sur le calibreur pour image 3D (1) et les points marqués sur l'image est le suivant :
①. Le groupage des points marqués sur le calibreur pour image 3D (1) en un groupe A et un groupe B, dans lequel chaque groupe comprend trois points marqués ou plus ;
②. La lecture des informations sur les points marqués compris dans le groupe A et le groupe B à l'étape
① et des informations sur le calibreur pour image 3D (1), et la lecture des images obtenues par scannage à l'étape 1) ;
③. La segmentation de seuil sur les images obtenues à l'étape ② et l'extraction et la production de polygones valables ;
④. L'ajustement et la détermination, selon les informations sur le calibreur pour image 3D (1) obtenues à l'étape ②, des polygones obtenus à l'étape ③, afin de sélectionner et obtenir les points marqués sur l'image ;
⑤. Le calcul d'une distance entre deux points marqués parmi les points marqués sur l'image obtenue à l'étape ④;
⑥. La sélection de trois points marqués parmi les points marqués du calibreur dans le groupe A pour construire un triangle comme modèle triangulaire, et la recherche d'un triangle approximativement identique ou équivalent au modèle triangulaire sur l'image ; si aucun triangle de la sorte n'est trouvé, la sélection de trois points marqués parmi les points marqués du calibreur dans le groupe B pour construire un triangle comme modèle triangulaire, et la recherche d'un triangle approximativement identique ou équivalent au modèle triangulaire sur l'image ; et s'il n'y a toujours pas de triangle de la sorte, la sélection de points marqués du calibreur dans le groupe A et le groupe B pour construire un triangle comme modèle triangulaire, et la recherche d'un triangle approximativement identique ou équivalent au modèle triangulaire sur l'image ; et
⑦. La rétention du nombre vertex de paires de triangles congruents selon une correspondance un-sur-un, pour former une paire de points correspondants, et la recherche des points marqués de l'image correspondante aux points marqués d'un calibreur en dehors du modèle triangulaire, sur l'image en utilisant le modèle de triangles congruents pour référence, jusqu'à ce que tous les points marqués de l'image correspondent aux points marqués du calibreur.
